(19) Europäisches Patentamt
European Patent Office
Office européen des brevets

(11) Veröffentlichungsnummer: **0 022 492**
**B1**

# (12) EUROPÄISCHE PATENTSCHRIFT

(45) Veröffentlichungstag der Patentschrift: **29.09.82**

(51) Int. Cl.³: **C 12 P 13/04**, C 07 B 19/00, C 07 C 103/46

(21) Anmeldenummer: **80103582.5**

(22) Anmeldetag: **25.06.80**

## (54) Stereoselektive Spaltung von Phenylglycinderivaten mit Enzymharzen.

(30) Priorität: **07.07.79 DE 2927535**

(43) Veröffentlichungstag der Anmeldung: **21.01.81 Patentblatt 81/3**

(45) Bekanntmachung des Hinweises auf die Patenterteilung: **29.09.82 Patentblatt 82/39**

(84) Benannte Vertragsstaaten: **AT BE CH DE FR GB IT LI NL SE**

(56) Entgegenhaltungen:
**EP-A-0 003 786**
**DE-A-2 419 838**
**DE-A-2 526 594**
**FR-A-1 012 740**
**FR-A-2 019 083**
**FR-A-2 215 465**
**FR-A-2 374 279**
**US-A-2 924 555**

**CHEMICAL ABSTRACTS, Band 88, Nr. 3, 16. Januar 1978, Zusammenfassung Nr. 20514r, Seite 446, Columbus, Ohio, US, W. HALWACHS et al.: "Application of immobilized chymotrypsin in a multistage fluidized-bed reactor"**

(73) Patentinhaber: **BAYER AG, Zentralbereich Patente, Marken und Lizenzen, D-5090 Leverkusen 1, Bayerwerk (DE)**

(72) Erfinder: **Schutt, Hermann, Dr., Gellertweg 12, D-5600 Wuppertal 1 (DE)**

(56) Entgegenhaltungen:
**CHEMICAL ABSTRACTS, Band 76, Nr. 15, 10. April 1972, Zusammenfassung Nr. 82748f, Seite 170, Columbus, Ohio, US, T.N. PATTABIRAMAN et al.: "Comparative studies of the specificities of alpha-chymotrypsin and subtilisin BPN'. Flexible substrates"**

**EP 0 022 492 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Stereoselektive Spaltung von Phenylglycinderivaten mit Enzymharzen

Die Erfindung betrifft ein Verfahren zur stereoselektiven Spaltung von DL-Phenylglycinderivaten durch Hydrolyse der Ester- oder Amidgruppen an N-Acyl-L-phenylglycinestern oder -amiden in N-Acyl-DL-phenylglycinestern oder -amiden durch Einwirken von Enzymen, Trennung der N-Acyl-D-phenylglycinester oder -amide von den N-Acyl-L-phenylglycinen und gegebenenfalls anschliessende saure Hydrolyse der Ester- oder Amidgruppen der D-Enantiomeren sowie der Acylgruppen, dadurch gekennzeichnet, dass man auf die N-Acyl-DL-phenylglycinester oder -amide in einem zweiphasigen Gemisch aus Lösungsmittel und Wasser trägergebundene Enzyme einwirken lässt.

D-Phenylglycin und D-4-Hydroxyphenylglycin dienen als Ausgangsstoffe für die Herstellung halbsynthetischer Antibiotika der Penicillin-Reihe. L-Phenylglycin ist Ausgangsstoff für den als Süssstoff verwendeten L-Asparagin-L-phenylglycin-methylester.

Die industriell eingeführte Methode zur Racemat-Spaltung von DL-Phenylglycin und Dl-4-Hydroxyphenylglycin ist die fraktionierte Kristallisation der Salze beider Aminosäuren mit DL-Campfersulfonsäure [J.P. Greenstein und M. Winitz, Chemistry of Amino acids, Vol. 1 (1961) 658]. Wegen des hohen Preises der Campfersulfonsäure muss diese möglichst vollständig wiedergewonnen werden, was technisch kaum zu realisieren ist. Es hat daher nicht an Versuchen gefehlt, Racemat-Spaltungen von Derivaten des Phenylglycins und des 4-Hydroxyphenylglycins auf völlig anderer Basis als der fraktionierten Kristallisation diastereoisomerer Verbindungen durchzuführen. Die neueren Verfahren versuchen die hohe Stereospezifität bestimmter Enzyme auszunutzen. Bei den bisher bekanntgewordenen Verfahen müssen jedoch eine Reihe von Nachteilen in Kauf genommen werden.

In der DE-A 2526594 wird L-Phenylglycinamid in DL-Phenylglycinamid durch eine dazu geeignete Aminopeptidase zum L-Phenylglycin hydrolysiert und vom unveränderten D-Phenylglycinamid getrennt, wobei das verwendete Enzym Leucinaminopeptidase (EC 3.4.1.1) teilweise an einen Träger gebunden ist. Nach diesem Verfahren kann nur in hoher Verdünnung gearbeitet werden, da das Hydrolyseprodukt L-Phenylglycin wegen seiner Schwerlöslichkeit auf dem Enzym kristallisieren und so dessen Aktivitäten in kürzester Zeit zum Erliegen bringen würde. Die Folge ist, dass grosse Volumina entstehen, die zur Isolierung des gewünschten Produktes energieaufwendig konzentriert werden müssen. Entsprechendes gilt für die Verfahren der DE-OS 2621076 und GB-A 1369462, in denen aus Phenyl- bzw. 4-Hydroxyphenylhydantoinen oder aus N-Phenacetyl-DL-4-hydroxyphenylglycin L-Phenylglycin und L-4-Hydroxyphenylglycin entstehen.

Es ist weiterhin aus Biochem. J. (1972), 126, 645–657 bekannt, DL-2-Acetamido-2-phenylessigsäuremethylester als 3%ige wässrige Suspension mit trägerfreiem α-Chymotrypsin zu D-2-Acetamido-2-phenylessigsäuremethylester und L-2-Acetamido-2-phenylessigsäure zu spalten, wobei von den Produkten nur 60 bzw. 52,5% der Theorie erhalten werden.

Es wurde nun überraschend gefunden, dass die erfindungsgemässe enzymatische Spaltung in wesentlich höherer Konzentration durchgeführt werden kann, wenn man die enzymatische Hydrolyse an trägergebundenen-proteolytischen Enzymen in zweiphasigen Wasser/Lösungsmittelgemischen durchführt.

Die zu spaltenden Verbindungen entsprechen insbesondere der Formel (I)

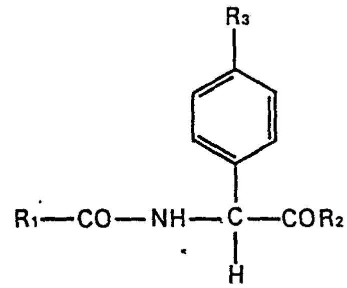

(I)

DL-Form

worin

$R_1$ Wasserstoff, einen gegebenenfalls substituierten aliphatischen Rest, insbesondere den Rest einer gegebenenfalls substituierten aliphatischen oder araliphatischen Mono- oder Dicarbonsäure oder einer natürlichen oder synthetischen α-Aminocarbonsäure,

$R_2$ Alkoxy oder gegebenenfalls durch Alkyl mono- oder disubstituiertes Amino oder den Rest einer natürlichen oder synthetischen α-Aminosäure und

$R_3$ Wasserstoff, Hydroxy, Alkoxy, Aralkoxy, Aryloxy, Cycloalkyloxy oder Acyloxy bedeuten.

Gegebenenfalls substituierte aliphatische Reste $R_1$ sind vor allem gegebenenfalls durch Halogen, insbesondere Fluor oder Chlor substituierte $C_1$–$C_9$-Alkyl- bzw. $C_2$–$C_9$-Alkenyl-Gruppen, vorzugsweise gegebenenfalls ein- bis dreimal durch Fluor oder Chlor substituierte $C_1$–$C_4$-Alkyl- bzw. $C_2$–$C_4$-Alkenylgruppen; araliphatische Reste $R_1$ sind insbesondere Phenyl-$C_1$–$C_2$-alkylgruppen, vorzugsweise der Benzylrest; die Reste $R_1$ und $R_2$, die sich von α-Aminosäure ableiten, sind insbesondere die von natürlichen α-Aminosäuren. Alkoxygruppen $R_2$ sind insbesondere $C_1$–$C_4$-Alkoxy, vorzugsweise Methoxy und Äthoxy. Gegebenenfalls durch Alkyl mono- oder disubstituierte Aminogruppen sind vorzugsweise Amino, Methyl-, Ethyl-, Dimethyl- und Diethylamino. Alkoxy $R_3$ ist insbesondere $C_1$–$C_4$-Alkoxy; Aralkoxy $R_3$ ist insbesondere Benzyloxy; Aryloxy $R_3$ ist insbesondere Phenyloxy; Cycloalkoxy $R_3$ ist insbesondere $C_5$- und $C_6$-Cycloalkoxy; Acyloxy $R_3$ ist insbesondere

gegebenenfalls durch 1-3 Fluor oder Chlor substituiertes $C_1$–$C_4$-Alkylcarbonyloxy.

Bevorzugte Verbindungen der Formel I sind solche, in denen $R_1$ Wasserstoff, Methyl, Mono-, Di- und Trichlormethyl, Trifluormethyl oder α-Carboxy-$C_2$–$C_6$-alkyl, $R_2$ Methoxy oder Ethoxy und $R_3$ Wasserstoff, Hydroxy, Methoxy, Ethoxy oder Acetoxy bedeuten.

Als Lösungsmittel für das Verfahren kommen mit Wasser nicht mischbare Lösungsmittel, wie Methylenchlorid, Chloroform, Toluol, Benzol, Essigsäureethylester, Petrolether, Methylisobutylketon oder Isobutanol in Frage.

Als Enzyme kommen insbesondere proteolytische Enzyme in Frage, insbesondere Serin- und Sulfhydryl-Proteasen, vorzugsweise Subtilisin (EC 3.4.4.16), α-Chymotrypsin (EC 3.4.4.5.), Papain (EC 3.4.4.10.), Ficin oder Bromelain, wobei die ersten beiden einen Serinrest im aktiven Zentrum der Aminosäurekette haben, während letztere in der Aminosäurekette Cystein als aktives Zentrum aufweisen. Subtilisin ist bevorzugt.

Das trägergebundene Enzym kann wenigstens 75mal mit nur geringem Verlust der Aktivität von ca. 0,1% pro Ansatz wiederverwendet werden, während nach dem Verfahren aus Biochem. J. lösliches Enzym überhaupt keinen Umsatz in Gegenwart von mit Wasser nicht mischbaren Lösungsmitteln zeigt.

Besondes geeignet sind aus Bacillus subtilis und Bacillus licheniformis isoliertes proteolytisches Subtilisin, das den Waschmitteln zur Entfernung von Proteinresten zugesetzt wird. Dieses technische Enzym ist vornehmlich unter den Firmennamen Maxatase® (Hersteller: Gist-Borcades N.V., Delft/Niederlande), Optimase® (Hersteller: Miles-Kali-Chemie, Hannover) und Alcalase® (Hersteller: Novo Industri AS, Kopenhagen/Dänemark) bekannt.

Die Eigenschaften der proteolytischen Enzyme, insbesondere ihre biochemischen Wirkungen, sind in folgenden Literaturstellen beschrieben: G.E. Perlmann und L. Lorand, Methods in Enzymology, 19 (1970), 199-215; P. Desnuelle, The Enzymes, 4 (1960), 93 und G.E. Perlmann und L. Lorand, Methods in Enzymology, 19 (1970), 226-244.

Die proteolytischen Enzyme können durch eine covalente Bindung über einen zur Katalyse nicht essentiellen Lysinrest an den polymeren Träger gekuppelt werden. Eine weitere Möglichkeit ist, die Adsorption des Enzyms an die Poren eines geladenen Trägers sowie die anschliessende Quervernetzung mit Glutardialdehyd.

Als Enzymträger kommen polymere, poröse Träger wie Cellulosen, z.B. DEAE- oder CM-Cellulosen, modifizierte Polyacrylamidgele mit Amino- oder Hydroxylgruppen oder verschiedene organische Copolymerisate aus Acrylamid, Methacrylaten oder Methacrylamid und Maleinsäureanhydrid nach den DE-A 2215539 und 2215687 in Frage.

Voraussetzung zur Eignung eines Enzymträgers für das beschriebene Verfahren ist ein vorheriger Test zur Adsorption von N-Acyl-DL-phenylglycinestern oder -amiden. Geeignete Träger dürfen N-Acyl-DL-phenylglycinester oder -amide nur in ganz geringem Umfang oder gar nicht adsorbieren.

Als besonders gute Träger für die proteolytischen Enzyme haben sich die verschiedenen Cellulosen, derivatisierten Cellulosen, Amino- oder Hydroxylgruppen-haltigen Polyacrylamidgele sowie die durch Amino- oder Hydroxylgruppen modifizierten Anhydridharze erwiesen. Generell können als Träger alle Amino- und Hydroxylgruppen tragenden polymeren Träger eingesetzt werden, die N-Acyl-Phenylglycinester oder -amide nicht adsorbieren.

Der polymere Träger wird nach an sich bekannten Methoden mit Cyanurchlorid [GB 1302706, N.L. Smith und H.M. Lehnhoff, Anal. Biochem. 61 (1974), 392-415, T.H. Finley, V. Troll, M. Levy, A.J. Johnson, L.T. Hodgins, Anal. Biochem. 87 (1978), 77-90] oder verschiedenen Halogenpyrimidinen nach DE-OS 2619521 und DE-OS 2619451 aktiviert.

Das Enzym wird zur Kupplung mit dem polymeren Träger unter optimalen Bedingungen für die Stabilität des Enzyms zur Reaktion gebracht. Die Effektivität der Kupplung kann durch Messung der enzymatischen Aktivität am Polymeren und im Waschwasser bestimmt werden. Das trägergebundene Enzym kann bei Verwendung im Batch-Verfahren leicht durch Sedimentation oder Filtration von der Reaktionslösung abgetrennt und mehrfach eingesetzt werden. Das trägergebundene Enzym kann auch in Säulen gefüllt und in Gegenwart eines Puffersystems von Substratlösung durchströmt werden.

Die in dem erfindungsgemässen Verfahren eingesetzten N-Acylester und -amide der Phenylglycine werden durch Acylierung der entsprechenden Aminosäureester- bzw. -amidhydrochloride mit stöchiometrischen Mengen Säureanhydrid wie Acetanhydrid und anschliessende Abtrennung des N-Acylderivates aus der wässrigen Phase mit organischen Lösungsmitteln wie Chloroform oder Methylenchlorid gewonnen.

Zahlreiche Substrate und Produkte von Enzymreaktionen sind in Wasser oder Pufferlösungen nur begrenzt löslich. Aus wirtschaftlichen Gründen sollten aber für die technische Anwendung von Reaktionen mit trägergebundenen Enzymen möglichst konzentrierte Substratlösungen eingesetzt werden.

Es wurden daher zahlreiche Versuche mit Wasser/Lösungsmittelgemischen unternommen, um mit Enzymen höhere Produktkonzentrationen als in wässriger Lösung erzielen zu können.

Die Beobachtung zeigt, dass gelöste, solvatisierte Enzyme durch Lösungsmittel, wie Methanol, Ethanol, Aceton, Acetonitril, Dioxan und Dimethylformamid oder Dimethylsulfoxyd teilweise oder vollständig unter partiellem oder vollständigem Verlust der Enzymaktivität denaturiert und aus der Lösung ausgefällt werden können.

Durch Bindung des Enzyms an einen polymeren Träger wird die Aggregation der Enzymmoleküle verhindert, so dass trägergebundene Enzyme in wesentlich geringerem Umfang als die freien Enzyme ihre Aktivität [K. Tamizawa und M.L. Bender, J. Biol. Chem. 249 (1974), 2130-2134] verlieren. Dennoch wird auch bei trägergebundenen Enzymen ein mehr oder minder starker Aktivitätsverlust des gebundenen Enzyms durch das Lösungsmittel beobachtet. Es wird entweder eine irreversible oder eine reversible Inaktivierung des Enzyms in Abhängigkeit von der Konzentration des Lösungsmittels beobachtet [H. Kaplan und K.J. Leidler, Canad. J. Chem. 45 (1967), 547-557, G.M. Umezurike, Biochem. J. 167 (1977), 831-833, T.N. Pattabiraman und W.B. Lawson, Biochem. J. 126 (1972), 645-657, G. Fink und H. Thoma, DECHEMA-Monographie 71, 295-314, H. Wan und C. Horvath, Biophys. Biochem. Acta 410 (1973), 135-140).

Die Enzymaktivität kann auch durch Veränderung der Porengrösse des Enzymträgers als Folge des Lösungsmitteleinflusses verändert werden.

Die oben erwähnten vollständig mit Wasser mischbaren Lösungsmittel weisen jedoch den weiteren technischen Nachteil auf, destillativ schwierig von Wasser abtrennbar zu sein.

Die Verwendung von mit Wasser nicht mischbaren Lösungsmitteln in Reaktionen mit trägergebundenen Enzymen ist bisher nur in einem Fall in der Literatur beschrieben [A.M. Klibanov, G.P. Samokhin, K. Martinek und I.V. Berezin, Biotechnol. Bioeng. 19 (1977) 1351-1361)]. In der zitierten Arbeit wird die Synthese von N-Acetyl-L-tryptophanethylester aus N-Acetyl-L-tryptophan und Ethanol mit kovalent an Glas gebundenem Chymotrypsin in Chloroform als Lösungsmittel beschrieben. Um die aus energetischen Gründen ungünstige Synthese des Esters durchführen zu können, muss in Abwesenheit von Wasser gearbeitet werden. Diese Literaturstelle gibt daher keinerlei Hinweis auf das erfindungsgemässe Verfahren.

Das erfindungsgemässe Verfahren vermeidet alle genannten Nachteile, schützt überdies die N-Acyl-phenylglycinester vor unspezifischer Hydrolyse, so dass die enzymatische Spaltung auch bei höheren pH-Werten ohne Einbusse an Ausbeute durchgeführt werden kann.

Gegen die denaturierende Wirkung von Lösungsmitteln kann das trägergebundene Enzym nach kovalenter Bindung an einem polymeren Träger durch nachträgliche inter- und intramolekulare Quervernetzung mit bi- oder polyvalenten Reagenzien wie Glutardialdehyd oder anderen Reagenzien (F. Wold, Methods in Enzymology II, 617-640) geschützt werden.

Die enzymatische Spaltung der N-Acyl-DL-phenylglycinester erfolgt vorzugsweise bei einer Temperatur von 20-40°C in einem pH-Bereich von 6-8, wobei der pH-Wert vorzugsweise durch Zugabe einer starken Base bei 7,0 konstant gehalten wird. Das Substrat wird als ca. 10-20%ige organische Lösung zu dem in Wasser suspendierten Enzym-Träger zugesetzt, wobei der Lösungsmittelanteil bezogen auf das Gesamtvolumen maximal 75-80 Volumen-% betragen kann. Das Reaktionsmedium wird während der enzymatischen Umsetzung intensiv gerührt. Der Verlauf und der Endpunkt der enzymatischen Reaktion kann durch die Neutralisation der entstehenden $H^+$-Ionen bestimmt werden. Die Neutralisation kann durch anorganische Basen als auch durch organische Basen erfolgen.

Nach Beendigung der enzymatischen Reaktion wird nach dem Absitzen des Enzymharzes die organische Phase abgenommen und die wässrige Rekationslösung portionsweise noch einmal mit dem zweifachen Volumen an organischen Lösungsmittel extrahiert. Die Extrakte werden vereinigt. Das Enzymharz wird abfiltriert und die verbleibende wässrige Phase, beispielsweise mit Schwefelsäure sauer gestellt und mit Essigester extrahiert. Die erhaltenen Verbindungen werden dann auf ihre optische Reinheit überprüft.

Die saure Hydrolyse zu den D- bzw. L-Phenylglycinen wird durch mehrstündiges (2-48 Stunden) Erhitzen (60-100°C) in Mineralsäuren, z.B. in 2n Salzsäure, durchgeführt. Nach dem Abkühlen der Lösung wird diese mit Natronlauge oder wässrigem Ammoniak auf pH 5-8 eingestellt und auf +4°C abgekühlt. Die auskristallisierten Phenylglycine werden abfiltriert, gewaschen und getrocknet.

Es ist bereits bekannt geworden, dass mikrobielle und tierische Serinproteasen wie Carlsberg- und Novo-Subtilisine oder Chymotrypsin einige N-Acyl-L-aminosäureester spalten können [A.O. Barel und A.N. Glazer, J. Biol. Chem. 243 (1968), 1344-1348 sowie US-A 3963573 und US-A 3878043]. Diese unterscheiden sich jedoch von den erfindungsgemäss zu spaltenden Derivaten des Phenylglycins erheblich. Nach Chemical Reviews 46 (1950), 69-153, insbesondere 119-122, waren ähnliche Ergebnisse bei acylierten Phenylglycinestern nicht zu erwarten.

Beispiel 1

200 g Cellulose Avicel (Merck) werden in einer Lösung aus 500 ml Wasser und 500 ml Dioxan suspendiert und mit 20 g Cyanurchlorid versetzt. Der pH-Wert wird mit 2 N NaOH zwischen pH 7,0 und 9,0 gehalten. Nach 45 Minuten Rühren wird die aktivierte Cellulose über eine Fritte abgesaugt und in 800 ml Wasser suspendiert. Es werden 25 g Maxatase (Gist-Brocades N.V., Delft/Niederlande) hinzugefügt und es wird 20 Stunden bei Raumtemperatur und pH 7-8 gerührt. Danach wird der Enzymträger über eine Fritte abgesaugt, portionsweise mit destilliertem Wasser gewaschen und zuletzt trocken gesaugt.

Es werden 600 g feuchte Subtilisin-Cellulose erhalten. Aktivität: 284 ATEE (N-Acetyl-L-tyrosinethylester)-Einheiten/g Enzymträger. Gesamtaktivität: 170250 ATEE-Einheiten, entsprechend 27,2% der eingesetzten Aktivität.

Beispiel 2

100 g DEAE-Cellulose (DE-52-Cellulose, Fa. Whatmann Ltd., Springfield/England) werden in ähnlicher Weise wie oben beschrieben mit Cyanurchlorid aktiviert. Davon werden 5 g nach Dialyse gegen Wasser lyophilisierte Maxatase oder

Alcalase (659 Anson-Einheiten/g, Hersteller Novo AS, Kopenhagen/Dänemark) kovalent gebunden. 140 g feuchtes DE-52- Cellulose-Subtilisin mit 740 g ATEE-Einheiten/g Enzymträger werden nach Filtration erhalten.

Die gesamte Aktivitätsausbeute betrug 103,600 ATEE-Einheiten entsprechend 16,5% der eingesetzten Aktivität.

Beispiel 3

20 g mit Aceton gewaschenes Anhydridharz (80 Gew.-% Tetraethylenglykoldimethacrylat, 10 Gew.-% Methacrylsäure und 10 Gew.-% Maleinsäureanhydrid) werden in 50 ml Wasser suspendiert. Es werden 40 ml 10 Gew.-%ige Hexamethylendiamin-Lösung bzw. Ethanolamin-Lösung bei pH 7,0 zugegeben und die Suspension über Nacht bei pH 6,2 durch Titration konstant gehalten. Das Aminharz wird abgesaugt. Mit 1 M NaCl-Lösung wird überschüssiges Hexamethylendiamin ausgewaschen. Anschliessend wird mit entsalztem Wasser gewaschen.

Das Aminogruppen tragende Anhydridharz wird in 50 ml Wasser und 50 ml Dioxan suspendiert und 1 Stunde bei Raumtemperatur mit 2 g Cyanurchlorid bei pH 5,0 aktiviert. Das Harz wird mit Dioxan und Wasser gewaschen und 20 Stunden bei Raumtemperatur mit 2 g Maxatase bei pH 8,0 umgesetzt.

Es werden 48,2 g feuchtes Harz mit einer Aktivität von 126 ATEE-Einheiten/g Enzymträger erhalten.

Die gesamte Aktivitätsausbeute betrug 60.790 ATEE-Einheiten entsprechend 12,1% der eingesetzten Aktivität.

Beispiel 4

300 g mit Diethylentriamin umgesetztes Polyacrylnitrilharz wird wie oben angegeben im gewichtsmässigen Verhältnis Träger : Cyanurchlorid (10 : 1) aktiviert und mit 30 g Maxatase bei 25°C, pH 8,0 und 16 Stunden zur Umsetzung gebracht.

Es werden 288,5 g feuchtes Enzymharz erhalten.

Die proteolytische Aktivität am Träger betrug 81,0 ATEE-Einheiten/g Enzymharz, insgesamt 23.383 ATEE-Einheiten entsprechend 3,1% der insgesamt eingesetzten Aktivität.

Beispiel 5

Herstellung von löslichem, trägergebundenem Subtilisin

20 g Stärke nach Zulkowsky werden in 500 ml Wasser gelöst, mit 2 N NaOH auf pH 11,0 eingestellt und mit 1,64 g Bromcyan versetzt. Zur Aktivierung des löslichen polymeren Trägers wird 30 Min. bei pH 11,0 am pH-Staten unter Zugabe von 2 N NaOH aktiviert. Der pH-Wert wird danach mit 2 N HCl auf pH 7,0 erniedrigt, 2 g dialysierte und lyophilisierte Maxatase zugefügt und die Lösung bei +4°C über Nacht gerührt.

Der Anteil von Maxatase, der nicht kovalent an Stärke gebunden wurde, wird über ein DDS-600-Ultrafilter entfernt.

Eingesetzte Subtilisin-Lösung: 60 625 ATEE-Einheiten.

Konzentrat nach Ultrafiltration = 46 400 ATEE-Einheiten = 76,5% der Gesamtaktivität.

Ultrafiltrat = 1500 ATEE-Einheiten = 2,5% der Gesamtaktivität.

Beispiel 6 (Vergleich)

Enzymatische Spaltung von N-Formyl-DL-phenylglycinmethylester sowie anschliessende saure Hydrolyse zu D-Phenylglycin ohne mit Wasser nicht mischbarem Lösungsmittel.

30 g N-Formyl-DL-phenylglycinmethylester werden in 2 l Wasser gelöst und mit 600 g nach Beispiel 1 hergestelltem Subtilisin-Träger unter Rühren versetzt. Der pH-Wert wird durch Zugabe von 25%igem Ammoniak mit einer Methrom-Titrationseinheit (pH-Wert E 300 B, Impulsomat E 473 und Dosimat 412, Methrom, Herisau/Schweiz) konstant bei pH 7,0 gehalten. Nach einer Reaktionszeit von 15 Stunden bei 37°C wird der Subtilisin-Träger über eine Fritte abgesaugt und bis zur weiteren Verwendung bei 4°C gelagert. Die Spaltungslösung wird in einem Rotationsverdampfer auf 500 ml eingeengt und portionsweise mit 200 ml, 100 ml und noch einmal 100 ml Methylenchlorid extrahiert. Die vereinigten Methylenchlorid-Extrakte werden im Rotationsverdampfer zur Trockene eingedampft.

Die Ausbeute an N-Formyl-D-phenylglycinmethylester beträgt 8,3 g (55,3% d. Theorie) bezogen auf 50% des racemischen Derivates

$$[\alpha]^{25°C}_{578\,nm} = -200{,}0°C\ (c = 1 \text{ in Methanol})$$

10 g N-Formyl-D-phenylglycinmethylester werden als 10%ige Lösung in 5%iger HCl gelöst und 24 Stunden bei 85°C hydrolysiert. Anschliessend wird die Salzsäure im Rotationsverdampfer abgezogen und das Konzentrat mit 15%iger Natronlauge auf pH 4,5 eingestellt. Der erhaltene Niederschlag wird abgesaugt, mit destilliertem Wasser und Aceton gewaschen und getrocknet.

Ausbeute an D-Phenylglycin = 7,22 g (92,2% der Theorie) bezogen auf N-Formyl-D-phenylglycinmethylester

$$[\alpha]^{25°C}_{578\,nm} = -158°C\ (c = 1 \text{ in } 1\ N\ HCl)$$

Beispiel 7

Enzymatische Spaltung von N-Acetyl-DL-phenylglycinmethylester in Gegenwart von Methylisobutylketon sowie anschliessende saure Hydrolyse zu D-Phenylglycin. 30 g N-Acetyl-DL-phenylglycinmethylester werden in einer Emulsion aus 250 ml Wasser und 250 ml Methylisobutylketon gelöst und mit 288,5 g nach Beispiel 4 hergestelltem Subtilisin-Harz versetzt. Nach 7 Stunden Rühren bei 37°C und pH 7,0 wird der Rührer abgeschaltet und die organische Phase abgenommen. Anschliessend wird die wässrige Phase noch zweimal mit 250 ml Methylisobutylketon extrahiert. Die vereinigten organischen Phasen werden im Rotationsverdampfer zur Trockene eingedampft.

Die Ausbeute an N-Acetyl-D-phenylglycinmethylester beträgt 14,9 g (99,3% der Theorie)

$[\alpha]^{25°C}_{578\,nm} = -176{,}2°$ (c = 1 in Methanol)

14,9 g N-Acetyl-D-phenylglycinmethylester werden wie in Beispiel 5 beschrieben zu D-Phenylglycin hydrolisiert und aufgearbeitet.

Ausbeute an D-Phenylglycin = 7,9 g (82,4% der Theorie)

$[\alpha]^{25°C}_{578\,nm} = -164{,}2°$ (c = 1 in 1 N HCl)

Beispiel 8

Enzymatische Spaltung von N-Acetyl-DL-phenylglycinmethylester in Gegenwart von Chloroform sowie anschliessende saure Hydrolyse zu D-Phenylglycin.

30 g N-Acetyl-DL-phenylglycinmethylester werden in einer Emulsion aus 800 ml Wasser und 200 ml Methylenchlorid gelöst und unter Rühren mit 140 g nach Beispiel 2 hergestelltem Subtilisin-Träger versetzt. Die enzymatische Spaltung und die Aufarbeitung erfolgte wie in Beispiel 6.

Die Ausbeute an N-Acetyl-D-phenylglycinmethylester beträgt 12,75 g (85,0% der Theorie)

$[\alpha]^{25°C}_{578\,nm} = -172{,}7°$ (c = 1 in Methanol)

Die Hydrolyse von N-Acetyl-0D-phenylglycinmethylester zu D-Phenylglycin erfolgte wie in Beispiel 6 beschrieben.

Beispiel 9

Enzymatische Spaltung von N-Acetyl-DL-4-acetoxyphenylglycin-methylester sowie anschliessende saure Hydrolyse zu D- und L-4-Hydroxy-phenylglycin.

30 g N-Acetyl-DL-4-acetoxyphenylglycinmethylester werden in einer Mischung aus 1 l Wasser und 1 l Methylisobutylketon gelöst und bei pH 7,0 und 37°C mit 100 000 ATEE-Einheiten DE-52-Cellulose-Subtilisin gespalten. Der pH-Wert wird mit 25%igem Ammoniak 8 Stunden konstant bei pH 7,0 gehalten. Nach dem Ausschalten des Rührers wird die organische Phase abgenommen und die wässrige Phase noch zweimal mit je 1 l Methylisobutylketon ausgerührt. Die vereinigten organischen Phasen werden im Rotationsverdampfer zur Trockne eingedampft.

Die Ausbeute an N-Acetyl-D-4-hydroxyphenylglycinmethylester beträgt 13,84 g (92,3% der Theorie)

$[\alpha]^{25°C}_{578\,nm} = -180{,}6°$ (c = 1 in Methanol)

Die wässrige Phase wird mit Schwefelsäure auf pH 1,5 eingestellt und dreimal mit je 1 l Essigsäureethylester extrahiert.

Die Ausbeute an N-Acetyl-L-4-hydroxyphenylglycin beträgt 11,26 g (95,5% der Theorie)

$[\alpha]^{25°C}_{578\,nm} = +159{,}8°$ (c = 1 in Methanol)

2,5 g N-Acetyl-D-p-hydroxyphenylglycinmethylester werden als 10%ige Lösung in 5%iger HCl bei 85°C 48 Stunden hydrolysiert. Anschliessend wird die Lösung abgekühlt und der pH-Wert mit 25%igem Ammoniak auf 4–6 eingestellt. Das ausgefallene D-4-Hydroxylphenylglycin wird abfiltriert, auf der Nutsche mit Wasser gewaschen und getrocknet.

Die Ausbeute an D-p-Hydroxyphenylglycin beträgt 1,69 g (90,2% der Theorie)

$[\alpha]^{25°C}_{578\,nm} = -158{,}2°$ (c = 1 in 1 N HCl)

Beispiel 10

Asymmetrische Spaltung von N-Acetyl-DL-Phenylglycinmethylester mit löslichem, trägergebundenem Subtilisin

30 g N-Acetyl-DL-Phenylglycinmethylester werden bei Raumtemperatur in 500 ml Methylisobutylketon gelöst. Die organische Phase wird zu 970 ml nach Beispiel 5 hergestelltem löslichem trägergebundenem Subtilisin gegeben und die Emulsion 15 Stunden bei 37°C und pH 7,0 gerührt. Die organische Phase wird abgetrennt und zur Trockene eingedampft.

Ausbeute an N-Acetyl-D-Phenylglycinmethylester:
13,6 g (90,9% der Theorie)

$[\alpha]^{25°C}_{578\,nm} = -178{,}0°$ (c = 1 in Methanol)

Aus der wässrigen Phase kann das lösliche, polymere Subtilisin durch Ultrafiltration über im DDS-600-Ultrafilter wiedergewonnen oder aber die wässrige Phase kann ohne Isolierung des polymeren Enzyms mehrfach mit organischer Phase zur Racematspaltung eingesetzt werden.

**Patentansprüche**

1. Verfahren zur stereoselektiven Spaltung von DL-Phenylglcinderivaten durch Hydrolyse der Ester- oder Amidgruppe an N-Acyl-L-phenylglycinestern oder -amiden in N-Acyl-DL-phenylglycinestern oder -amiden durch Einwirkung von Enzymen, Trennung der N-Acyl-D-phenylglycinester oder -amide von den N-Acyl-L-phenylglycinen und gegebenenfalls anschliessende saure Hydrolyse der Ester- oder Amidgruppen der

D-Enantiomeren sowie der Acylgruppen, dadurch gekennzeichnet, dass man auf die N-Acyl-Dl-phenylglycinester oder -amide in einem zweiphasigen Gemisch aus Lösungsmittel und Wasser trägergebundene Enzyme einwirken lässt.

2. Verfahren nach Anspruch 1, dadurch gekennzeichnet, dass man als Substrat eine Verbindung der Formel einsetzt,

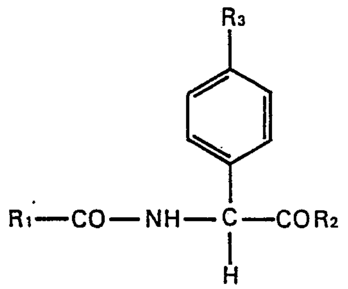

(I)

DL-Form

worin

$R_1$ Wasserstoff, einen gegebenenfalls substituierten aliphatischen Rest, insbesondere den Rest einer gegebenenfalls substituierten aliphatischen oder araliphatischen Mono- oder Dicarbonsäure oder einer natürlichen oder synthetischen α-Aminocarbonsäure,

$R_2$ Alkoxy oder gegebenenfalls durch Alkyl mono- oder disubstituiertes Amino oder den Rest einer natürlichen oder synthetischen α-Aminosäure und

$R_3$ Wasserstoff, Hydroxy, Alkoxy, Aralkoxy, Aryloxy, Cycloalkoxy oder Acyloxy bedeuten.

3. Verfahren nach Anspruch 2, worin

$R_1$ Wasserstoff, Methyl, Mono-, Di- oder Trichlormethyl, Trifluormethyl oder α-Carboxy-$C_2$–$C_6$-alkyl,

$R_2$ Methoxy oder Ethoxy und

$R_3$ Wasserstoff, Hydroxy, Methoxy, Ethoxy oder Acetoxy bedeuten.

4. Verfahren nach Anspruch 1, dadurch gekennzeichnet, dass man als Enzym Subtilisin, α-Chymotrypsin, Papain, Ficin oder Bromelain einsetzt.

5. Verfahren nach Anspruch 1, dadurch gekennzeichnet, dass man als wasser-nicht-mischbares Lösungsmittel Methylenchlorid, Chloroform, Toluol, Benzol, Essigsäureethylester, Petrolether, Methylisobutylketon oder Isobutanol einsetzt.

6. Verfahren nach Anspruch 1, dadurch gekennzeichnet, dass man als Träger des Enzyms Cellulosen, derivatisierte Cellulosen, amino- oder hydroxylgruppenhaltige Polyacrylamidgele oder durch Amino- oder Hydroxylgruppen modifizierte Anhydridharze einsetzt.

**Claims**

1. Process for the stereoselective resolution of DL-phenylglycine derivatives by hydrolysing the ester or amide groups of N-acyl-L-phenylglycine esters or amides in N-acyl-DL-phenylglycine esters or amides by the action of enzymes, separating the N-acyl-D-phenylglycine esters or amides from the N-acyl-L-phenylglycines and then, if appropriate, subjecting the ester or amide groups of the D-enantiomers and the acyl groups to acid hydrolysis, characterised in that enzymes which are bonded to carriers are allowed to act on the N-acyl-DL-phenylglycine esters or amides in a two-phase mixture consisting of solvent and water.

2. Process according to Claim 1, characterised in that a compound of the formula

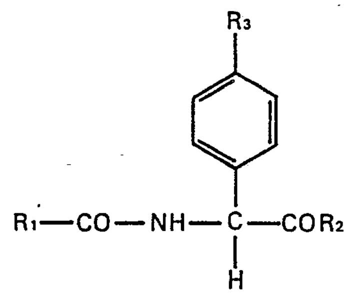

(I)

DL-Form

wherein

$R_1$ denotes hydrogen or an optionally substituted aliphatic radical, in particular the radical of an optionally substituted aliphatic or araliphatic monocarboxylic or dicarboxylic acid or of a naturally occurring or synthetic α-aminocarboxylic acid,

$R_2$ denotes alkoxy, amino which is optionally monosubstituted or disubstituted by alkyl or the radical of a naturally occurring or synthetic α-aminoacid and

$R_3$ denotes hydrogen, hydroxyl, alkoxy, aralkoxy, aryloxy, cycloalkoxy or acyloxy,

is used as the substrate.

3. Process according to Claim 2,

wherein

$R_1$ denotes hydrogen, methyl, mono-, di- or trichloromethyl, trifluoromethyl or α-carboxy-$C_2$–$C_6$-alkyl,

$R_2$ denotes methoxy or ethoxy and

$R_3$ denotes hydrogen, hydroxyl, methoxy, ethoxy or acetoxy.

4. Process according to Claim 1, characterised in that subtilisin, α-chymotrypsin, papain, ficin or bromelain is employed as the enzyme.

5. Process according to Claim 1, characterised in that methylene chloride, chloroform, toluene, benzene, ethyl acetate, petroleum ether, methyl isobutyl ketone or isobutanol is employed as the water-immiscible solvent.

6. Process according to Claim 1, characterised in that celluloses, cellulose derivatives, polyacrylamide gels containing amino groups or hydroxyl groups or anhydride resins modified by amino groups or hydroxyl groups are employed as the carrier for the enzyme.

**Revendications**

1. Procédé de dissociation stéréosélective de dérivés de DL-phénylglycine par hydrolyse des groupes ester ou amido sur des esters ou des

amides de N-acyl-L-phénylglycine en esters ou en amides de N-acyl-DL-phénylglycine sous l'action d'enzymes, séparation des esters ou des amides de N-acyl-D-phénylglycine des N-acyl-L-phénylglycines et éventuellement par hydrolyse acide ultérieure des groupes esters ou amido des D-énantiomères; ainsi que des groupes acyle, caractérisé en ce que, sur les esters ou les amides de N-acyl-DL-phénylglycine, on fait agir des enzymes fixées sur un support dans un mélange à deux phases d'un solvant et d'eau.

2. Procédé suivant la revendication 1, caractérisé en ce que, comme substrat, on utilise un composé de formule:

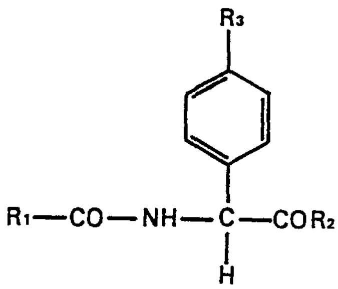

(I)

forme DL

dans laquelle

$R_1$ représente un atome d'hydrogène, un radical aliphatique éventuellement substitué, en particulier, le radical d'un acide monocarboxylique ou dicarboxylique araliphatique ou aliphatique éventuellement substitué ou d'un acide α-aminocarboxylique naturel ou synthétique,

$R_2$ représente un groupe alcoxy ou un groupe amino éventuellement monosubstitué ou disubstitué par un groupe alkyle, ou encore le radical d'un α-amino-acide naturel ou synthétique, et

$R_3$ représente un atome d'hydrogène, un groupe hydroxy, alcoxy, aralcoxy, aryloxy, cycloalcoxy ou acyloxy.

3. Procédé suivant la revendication 2, caractérisé en ce que:

$R_1$ représente un atome d'hydrogène, un groupe méthyle, monochlorométhyle, dichlorométhyle, trichlorométhyle, trifluorométhyle ou α-carboxy-alkyle en $C_2$–$C_6$,

$R_2$ représente un groupe méthoxy ou éthoxy, et

$R_3$ représente un atome d'hydrogène, un groupe hydroxy, méthoxy, éthoxy ou acétoxy.

4. Procédé suivant la revendication 1, caractérisé en ce que, comme enzyme, on utilise la subtilisine, l'α-chymotrypsine, la papaïne, la ficine ou la bromélaïne.

5. Procédé suivant la revendication 1, caractérisé en ce que, comme solvant non miscible à l'eau, on utilise le chlorure de méthylène, le chloroforme, le toluène, le benzène, l'ester éthylique d'acide acétique, l'éther de pétrole, la méthylisobutylcétone ou l'isobutanol.

6. Procédé suivant la revendication 1, caractérisé en ce que, comme support de l'enzyme, on utilise des celluloses, des dérivés de celluloses, des gels de polyacrylamides contenant des groupes amino ou hydroxy, ou encore des résines d'anhydrides modifiées par des groupes amino ou hydroxy.